# EUROPÄISCHE PATENTANMELDUNG

(11) **EP 4 706 365 A1**
(43) Veröffentlichungstag der Anmeldung: **11.03.2026**
(21) Anmeldenummer: 25183584.9
(22) Anmeldetag: 18.06.2025
(51) Int. Cl.: A01B 79/00, A01D 75/18, G01N 21/85, A01D 78/10

(54) **LANDMASCHINE MIT SCHWADSENSORIK**

(30) Priorität: 03.07.2024 DE 102024118936
(71) Anmelder: CLAAS Saulgau GmbH, 88340 Bad Saulgau (DE)
(72) Erfinder: Fischer, Josef, 88400 Biberach (DE); Becker, Michael, 88367 Hohentengen (DE); Neitemeier, Dennis, 59510 Lippetal (DE)
(74) Vertreter: CLAAS Gruppe

(57) **Zusammenfassung**

Eine Landmaschine (1) umfasst eine Schwadsensorik (9-12) zum Erfassen von einer oder mehreren Eigenschaften eines Schwads (8) auf einer von der Landmaschine (1) befahrenen landwirtschaftlichen Fläche (4). Die Landmaschine (1) umfasst ferner eine Vorrichtung zur Schwaderzeugung (3, 16), und wenigstens ein Sensor (10, 11, 12) der Schwadsensorik (9-12) ist angeordnet, um Eigenschaften eines Stroms von schwadbildendem Material während oder nach seiner Verarbeitung zum Schwad (8) durch die Vorrichtung (3, 16) zu erfassen

## Beschreibung

Die vorliegende Erfindung betrifft eine selbstfahrende oder mit Hilfe einer Zugmaschine bewegliche Landmaschine mit einer Schwadsensorik zum Erfassen von Eigenschaften eines Erntegutschwads.

Eine solche Landmaschine in Form eines Traktors, der eine Ballenpresse hinter sich herzieht, ist aus EP 3 365 649 B1 bekannt. Die Schwadsensorik umfasst hier einen Radarsensor, der an der Frontpartie des Traktors, etwa an einer Motorhaube oder der Vorderkante eines Kabinendachs, angebracht ist, um Eigenschaften eines Schwads zu erfassen, unmittelbar bevor dieser von der Ballenpresse aufgenommen wird.

Zu den von dieser herkömmlichen Schwadsensorik erfassten Eigenschaften kann die Feuchtigkeit des Schwads gehören. Anhand der gemessenen Feuchtigkeit kann entschieden werden, ob das Material des Schwads für eine geplante Verwendung geeignet ist oder nicht. Wenn sich allerdings herausstellt, dass das Material für die geplante Verwendung zu feucht oder zu trocken ist, dann ist zu diesem Zeitpunkt bereits Zeit und Energie investiert worden, um die Landmaschine aufs Feld zu bringen, und dieser Aufwand ist vergeblich, wenn die geplante Verarbeitung nicht durchgeführt werden kann.

Eine weitere Eigenschaft, die mit dieser herkömmlichen Schwadsensorik überwacht werden kann, ist das Vorhandensein von Fremdkörpern im Schwad. Insbesondere metallische Fremdkörper sollten nicht mit dem Schwad aufgenommen werden, da sie, wenn sie ins Viehfutter gelangen, zu Verletzungen führen können. Um dies zu vermeiden, muss der Traktor bei Erfassung eines Fremdkörpers rechtzeitig angehalten werden, damit der Fremdkörper beseitigt werden kann. Eine abrupte Bremsung ist nicht nur für den Fahrer unangenehm; sie kann auch den Boden schädigen, indem sie zur Verschiebung von Erde und zu Unebenheit führt. Bei behutsamerer Bremsung kann die Beseitigung des Fremdkörpers dadurch erschwert sein, dass der Traktor sich darüber befindet.

Anhand der Vermessung von geometrischen Eigenschaften des Schwads kann die Menge an im Schwad enthaltenem Material abgeschätzt und die zu seiner Bergung erforderliche Transportkapazität abgeschätzt werden, allerdings erst wenn die Verarbeitung des Schwads durch die Maschine bereits begonnen hat.

Aufgabe der vorliegenden Erfindung ist, wenigstens einen der oben genannten Nachteile zu beheben.

Die Aufgabe wird gelöst, indem eine Landmaschine mit einer Schwadsensorik zum Erfassen von einer oder mehreren Eigenschaften eines Schwads auf einer von der Landmaschine befahrenen landwirtschaftlichen Fläche, ferner eine Vorrichtung zur Schwaderzeugung umfasst und wenigstens ein Sensor dieser Schwadsensorik angeordnet ist, um Eigenschaften eines Stroms von schwadbildendem Material während oder nach seiner Verarbeitung durch die Vorrichtung zu erfassen.

Die Schwadsensorik ermöglicht also dadurch, dass sie an der den Schwad erzeugenden Maschine selbst vorgesehen ist, eine frühzeitige Erfassung aller interessierenden Eigenschaften.

Wenn etwa bereits zum Zeitpunkt der Schwaderzeugung dessen Feuchte gemessen wird, kann deren weitere Entwicklung anhand der Wetterbedingungen recht genau vorhergesagt und eine gewünschte Weiterverarbeitung zum frühestmöglichen Zeitpunkt durchgeführt werden, zu dem die Restfeuchte des Schwads dies zulässt.

Ein bereits zum Zeitpunkt der Schwadbildung erfasster Fremdkörper kann zu jedem geeigneten Zeitpunkt vor der Weiterverarbeitung des Schwads entfernt werden; selbst wenn er bis dahin nicht entfernt wird, kann in Kenntnis des Ortes des Fremdkörpers eine die Weiterverarbeitung vornehmende Maschine rechtzeitig vor Erreichen des Fremdkörpers angehalten werden, ohne dass dafür eine abrupte Bremsung erforderlich wird.

Anhand von bereits zum Zeitpunkt der Schwaderzeugung gewonnener Information über die in dem Schwad enthaltene Materialmenge kann für ausreichende Transportkapazität zum Abtransport des Materials gesorgt werden, ohne dass kurzfristig improvisiert werden muss.

Wenigstens ein Sensor der Sensorik kann auf einen in Fahrtrichtung der Landmaschine hinter der Vorrichtung liegenden Bereich der landwirtschaftlichen Fläche ausgerichtet sein, um Eigenschaften eines von der Vorrichtung erzeugten Schwads zu erfassen.

Als ein solcher Sensor kommt insbesondere ein berührungsloser Sensor wie etwa eine Kamera, ein Radarsensor, ein Lidarsensor oder ein NIR-Sensor in Betracht.

Zum Erfassen von Eigenschaften eines Stroms des schwadbildenden Materials noch vor Bildung des Schwads kommen noch weitere, insbesondere nicht berührungslose Sensortypen in Betracht. Wenn etwa die Vorrichtung zur Schwaderzeugung ein Kreiselschwader ist, kommt als Sensor etwa ein Nabensensor in Betracht, der in einer Nabe des Kreisels angeordnet ist, um ein Drehmoment zu erfassen, das von dem Kreisel beim Schwaden in Bewegung gesetztes Material auf den Kreisel ausübt, oder ein Schwadtuchsensor, der auf an ein Schwadtuch aufprallendes Material anspricht.

Die Sensorik sollte eingerichtet sein, Messdaten der ein oder mehreren Eigenschaften jeweils verknüpft mit einem Ort ihrer Messung zu liefern. Anhand dieser Daten kann ein Ort, an dem zur Zeit der Schwadbildung z.B. ein Fremdkörper im Schwad erfasst wurde, zu einem späteren Zeitpunkt wiedergefunden und der Fremdkörper beseitigt werden, oder es kann der Ort, an dem beim späteren Bergen des Schwads die Ladekapazität einer Bergemaschine erschöpft und ein Überladen von der Bergemaschine auf ein Transportfahrzeug notwendig ist, exakt vorhergesagt und durch bedarfsgerechte Bereitstellung des Transportfahrzeugs eine zügige Bergung durchgeführt werden.

Die ein oder mehreren Eigenschaften können wenigstens eine von Schwadhöhe, -breite, -querschnitt, Position einer Schwadober- oder -unterseite, Trockenmasse- oder Wasseranteil, Dichte, Homogenität, enthaltene Pflanzenarten betreffen. Messwerte zu den Abmessungen des Schwads und seiner Zusammensetzung können fusioniert werden, um eine Abschätzung der im Schwad enthaltenen Materialmenge zu gewinnen. Daten zu Dichte oder Homogenität ermöglichen die Identifizierung von Fremdkörpern, verklumptem, verfilztem oder zu einem Zopf verdrilltem Material, das Schwierigkeiten beim Bergen des Schwads oder der anschließenden Weiterverarbeitung bereiten kann.

Um die Beschaffenheit des Schwads exakt zu erfassen, kann die Schwadsensorik mehrere quer zur Fahrtrichtung gegeneinander versetzte Sensoren umfassen. Diese können jeweils dieselbe Messgröße, wie etwa eine Höhe oder Schichtdicke, an verschieden Orten des Schwadquerschnitts erfassen. Denkbar ist aber auch, mehrere insbesondere ortsauflösende Sensoren unterschiedlichen Typs so anzuordnen, dass sich ihre Erfassungsbereiche nur zum Teil überlappen; indem die Sensorik an im überlappenden Bereich gewonnener Daten beider Sensoren lernt, wie diese normalerweise kombiniert sind, kann sie für einen Bereich außerhalb des Überlappungsbereichs, für den Daten eines der Sensoren fehlen, anhand dort gemessener Daten wenigstens eines anderen Sensors die fehlenden Daten abschätzen.

Des Weiteren kann wenigstens ein Sensor angeordnet sein, um wenigstens eine Eigenschaft von zum Schwad seitlich benachbarten Teilen der Fläche zu erfassen. Solche Daten, etwa zu Oberflächenbeschaffenheit und -feuchte können hilfreich sein, um die Entwicklung des Feuchtegehalts des Schwads zu prognostizieren und einen Zeitpunkt der Bergung passend zu wählen. Sie können aber auch dazu dienen, die Schwaderzeugung zu steuern, etwa indem die Dichte von zum Bilden des Schwads geeignetem Material, das von der Vorrichtung zur Schwaderzeugung nicht erfasst worden ist und das deshalb seitlich vom Schwad liegengeblieben ist, erfasst wird und die dabei gewonnenen Messwerte zum Steuern der schwadbildenden Vorrichtung genutzt werden.

Weitere Merkmale und Vorteile der Erfindung ergeben sich aus der nachfolgenden Beschreibung von Ausführungsbeispielen unter Bezugnahme auf die beigefügten Figuren. Es zeigen:
- Fig. 1: eine Draufsicht auf eine Landmaschine gemäß einer ersten Ausgestaltung der Erfindung;
- Fig. 2: eine Ansicht einer Landmaschine gemäß einer zweiten Ausgestaltung; und
- Fig. 3: einen Sensorbalken der Landmaschine aus Fig. 1 oder Fig. 2.

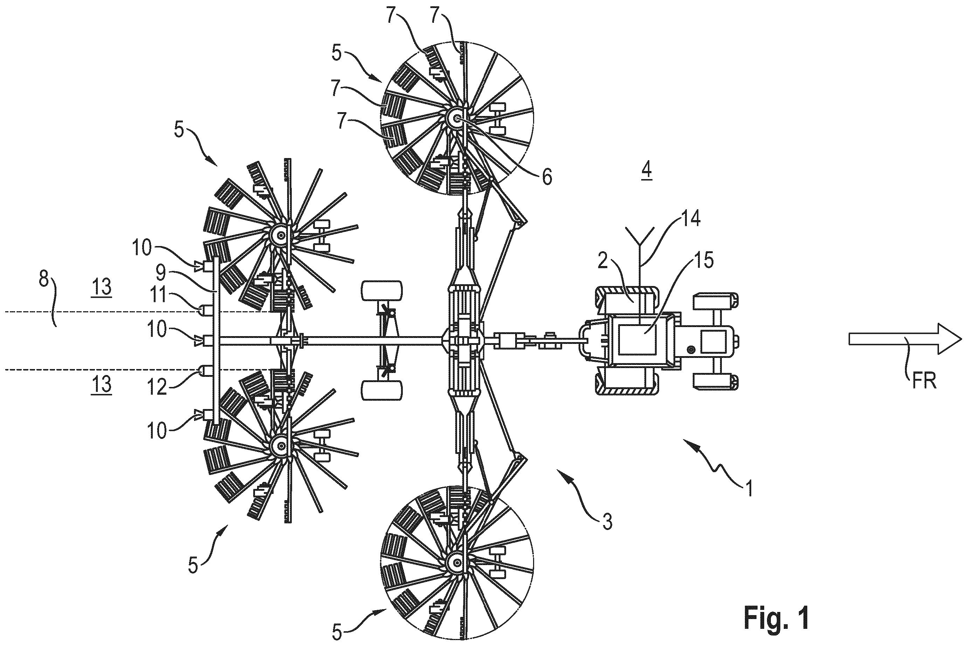

Fig. 1 zeigt in einer Draufsicht ein Gespann 1 aus einem Traktor 2 und einem von dem Traktor 2 gezogenen Schwader 3 auf einer landwirtschaftlichen Fläche 4. Der Schwader 3 umfasst mehrere, hier zwei, in Fahrtrichtung FR des Gespanns aufeinanderfolgende Paare von Kreiseln 5. Die Kreisel 5 sind in an sich bekannter Weise über eine Zapfwelle des Traktors 2 drehangetrieben und haben radial von einer Nabe 6 abstehende Rechen 7. Indem die Rechen 7 auf einem Teil ihres Umlaufs um die Nabe 6 gegen den Boden geschwenkt werden, erfassen sie dort liegendes Pflanzenmaterial und werfen es zur Mitte des Schwaders 3, so dass dort einem Schwad 8 zu formen, der hinter dem Gespann 1 auf der Fläche 4 liegenbleibt.

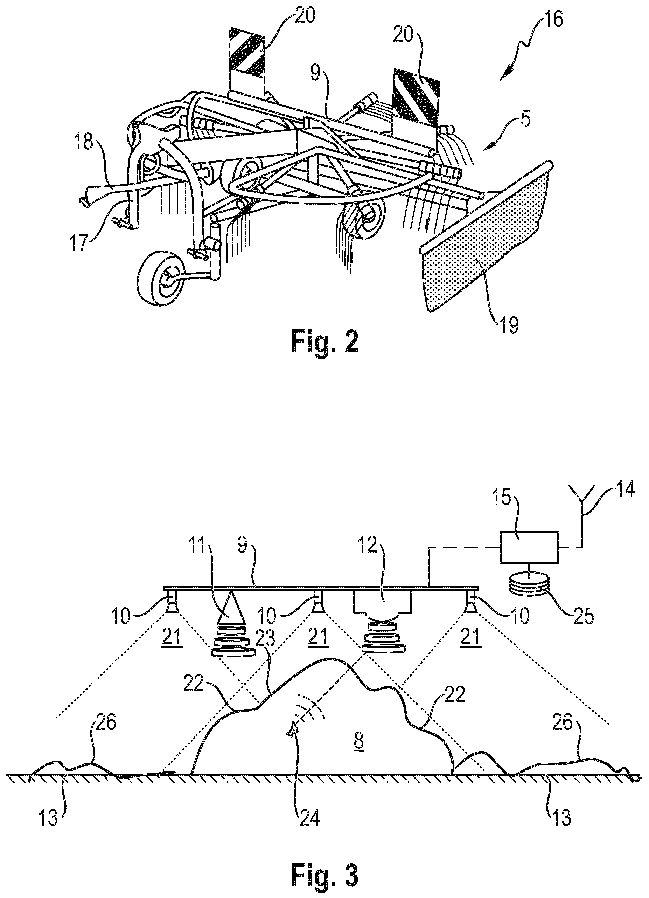

Am Heck des Schwaders 3 ist ein Sensorbalken 9 quer zur Fahrtrichtung montiert. Der Sensorbalken 9 trägt eine Mehrzahl von Sensoren 10, 11, 12, die auf den Schwad 8 und Bereiche 13 der Fläche 4 beiderseits des Schwads 8 ausgerichtet sind. Die Sensoren 10, 11, 12 werden später mit Bezug auf Fig. 3 genauer beschrieben.

Als weitere Sensoren können in der Fig. nicht erkennbare Nabensensoren von an sich bekannter Bauart an jeder Nabe 6 vorgesehen sein, um das dort zwischen Antrieb und Kreisel 5 wirkende Drehmoment zu erfassen.

Die Sensoren sowie ein Satellitennavigationssystem 14 kommunizieren mit einem Computer 15 an Bord des Traktors 2. Der Computer 15 kann im Traktor 2 fest installiert sein; es kann sich aber auch um einen mobilen Computer handeln, z.B. ein Notepad oder Smartphone, das von einem Fahrer des Traktors 2 mitgeführt wird.

Fig. 2 zeigt einen Schwader 16 mit einem einzelnen Kreisel 5 in einer Ansicht schräg von vorn, entgegen der Fahrtrichtung. An der Vorderseite des Schwaders 16 erkennt man ein Joch 17 zur Ankopplung an den Traktor 2 sowie eine Welle 18 zum Verbinden mit einer Zapfwelle des Traktors 2. Durch die Drehung des Kreisels 5 seitwärts beschleunigtes Material trifft hier auf ein in Fahrtrichtung langgestrecktes Schwadtuch 19 und bildet den (in Fig. 2 nicht dargestellten) Schwad, indem es an dem Schwadtuch 19 zu Boden fällt. Ein für die Menge des aufprallenden Materials empfindlicher Sensor ist in das Schwadtuch integriert. Die Struktur eines hierfür geeigneten kapazitiven Sensors ist in dem nicht vorveröffentlichten Dokument DE 10 2024 113 247.7 beschrieben.

Als Sensorbalken 9 kann hier ein Querträger dienen, an dem herkömmlicherweise Warnschilder 20 für den Straßenverkehr angebracht sind.

Fig. 3 zeigt den Sensorbalken 9 mit den daran verteilten Sensoren 10, 11, 12. Die Sensoren 10 sind Kameras. Die Kameras 10 können untereinander identisch sein oder sich in ihrer spektralen Empfindlichkeit unterscheiden. Während bei dem meisten elektronischen Kameras drei Sensortypen für die Farben Rot, Grün und Blau vorgesehen sind, deren spektrale Empfindlichkeit jeweils die der Sinneszellen des menschlichen Auge nachbildet, kann wenigstens eine der Kameras 10 auch eine abweichende Zahl von Sensortypen umfassen, darunter solche, die für Spektralbereiche im Infraroten oder Ultravioletten empfindlich sind oder Sensortypen, die schmalbandig empfindlich sind für charakteristische Farbtöne eines bestimmten Bestandteils des Schwads und deren Messsignal somit einen sicheren Rückschluss auf Vorhandensein bzw. Konzentration dieses Bestandteils im Schwad 8 erlaubt.

Raumwinkel 21, in denen die Kameras 10 empfindlich sind, sind in Fig. 3 durch punktierte Linien begrenzt. Oberflächenbereiche 22 des Schwads 8, die im Überschneidungsbereich der Raumwinkel 21 von zwei Kameras 10 liegen, können stereoskopisch vermessen werden. Der Abstand zwischen zwei Kameras 10 ist größer als bei den meisten stereoskopischen Messystemen, insbesondere kann er größer sein als der halbe Abstand der Kameras 10 vom Boden, so dass ein Bereich 23 der Schwadoberseite, der außerhalb des Überschneidungsbereichs der Raumwinkel 21 liegt, nicht stereoskopisch vermessen werden kann; der Verlauf der Oberseite in diesem Bereich 23 kann jedoch durch den Computer 15 anhand von Messwerten der benachbarten Bereiche 22 abgeschätzt werden.

Alternativ oder ergänzend können zur Abschätzung des Verlaufs der Schwadoberseite im Bereich 23 Daten der jeweils zwischen den Kameras 10 angeordneten Lidar- bzw. Radarsensoren 11, 12 herangezogen werden. Da diese Sensoren 11, 12 auf einer Laufzeitmessung basieren, können sie jeweils für sich allein Messdaten zum Oberseitenverlauf liefern; es müssen nicht wie bei den Kameras 10 Daten mehrerer Sensoren kombiniert werden, um die Höhe eines Punkts der Schwadoberseite über dem Boden berechnen zu können.

Des Weiteren kann insbesondere der Radarsensor 12, da die von ihm emittierte Strahlung in den Schwad 8 eindringt, weitergehenden Aufschluss über dessen Aufbau geben. Die Intensität eines von der Oberseite 23 sowie von Inhomogenitäten im Innern des Schwads 8 zurückgeworfenen Echos erlaubt einen Rückschluss auf die Dichte des Schwads 8; anhand eines Echos der Bodenoberfläche kann deren Höhe abgeschätzt werden, was für die Berechnung des Schwadquerschnitts und damit letztlich der im Schwad 8 enthaltenen Materialmenge von Bedeutung ist.

Anhand des von ihm erzeugten Radarechos ist die Position eines Fremdkörper 24 im Schwad ermittelbar.

Von den Kameras 10 gelieferte Farbinformation, ggf. unterstützt von auf Bildern der Kameras 10 basierender Mustererkennung, ermöglicht eine

Identifikation von im Schwad 8 enthaltenen Pflanzenarten, ihrem Anteil am Schwad 8 und dem Grad der Trocknung, was ebenfalls die Berechnung der Materialmenge beeinflusst.

Um Aufschluss über Eigenschaften des Schwads wie etwa die Zusammensetzung nach Arten oder Inhaltsstoffen, insbesondere den Gehalt an Wasser oder Nährstoffen, zu gewinnen, kann anstelle eines der Sensoren 11, 12 oder ergänzend dazu ein NIR-Sensor vorgesehen sein. Der NIR-Sensor unterscheidet sich von einer im NIR empfindlichen Kamera 10 dadurch, dass die Intensität der IR-Strahlung nicht über ein breites Spektralintervall integriert, sondern wellenlängenaufgelöst erfasst wird, was einen erheblich genaueren Rückschluss auf die chemische Beschaffenheit des untersuchten Materials erlaubt, dafür aber eine Ortsauflösung nur in stark eingeschränktem Umfang möglich ist, indem die Raumrichtung, aus der Strahlung aufgefangen und analysiert wird, optional mit Hilfe einer Abtastoptik verändert wird.

Während der Sensorbalken 9 während des Arbeitseinsatzes des Gespanns 1 über den frisch vom Schwader 3 oder 16 erzeugten Schwad 8 hinwegbewegt wird, sammeln dessen Sensoren 10, 11, 12 und ggf. die Nabensensoren, der Sensor des Schwadtuchs 19 und/oder der NIR-Sensor Daten über in der Fahrtrichtung FR aufeinanderfolgende Scheiben des Schwads 8. Diese Daten sind nicht unbedingt geeignet, um daraus nach einer deterministischen Formel den Materialinhalt einer solchen Scheibe zu berechnen; dennoch gibt es Kombinationen von Ausgangsdaten der diversen Sensoren, die in ihrer Kombination mit bestimmten Werten von Eigenschaften der Scheibe wie etwa Zusammensetzung, Dichte, Feuchtigkeitsgehalt und dergleichen zusammenhängen und in ihrer Kombination miteinander einen Rückschluss auf die geseuchten Parameter zulassen. Der Computer 15 kann programmiert sein, um während eines Einsatzes in einem Massenspeicher 25 lediglich die von den Sensoren gelieferten Datenwerte, jeweils verknüpft mit dem von dem Navigationssystem erfassten Ort ihrer Gewinnung, abzuspeichern, so dass eine Auswertung der so gesammelten Daten zu einem späteren Zeitpunkt, eventuell durch einen anderen, leistungsfähigeren Computer, stattfinden kann. Wenn der Computer 15 über eine ausreichende Rechenleistung verfügt, kann er eine Auswertung der Daten auch bereits während des Einsatzes vornehmen und in den Massenspeicher 25, zusammen mit den jeweiligen Orten der Messungen, ggf. zusammen mit den ursprünglich von den Sensoren an dem betreffenden Ort empfangenen Daten, auch die Ergebnisse der Auswertung, eintragen.

Die Auswertung kann darin bestehen, in den erfassten Sensordaten bekannte Wertekombinationen oder solche, die bekannten Kombinationen ähnlich sind, zu identifizieren und anhand von zuvor ermittelten, diesen Kombinationen entsprechenden Werten von Eigenschaften eines Schwads dem aktuellen Schwad 8 die entsprechenden Werte dieser Eigenschaften zuzuordnen. So kann z.B. für jede einem Satz von Sensordaten entsprechende Scheibe des Schwads eine Masse der Scheibe oder deren Platzbedarf auf dem zum Bergen verwendeten Fahrzeug abgeschätzt werden; diese Daten können wiederum dazu dienen, vorherzusagen, an welchen Stellen des von diesem Fahrzeug beim Bergen des Schwads 8 auf dem Feld zurückgelegten Weg ein Umladen des geborgenen Materials auf ein Transportfahrzeug nötig ist, und die Bewegungen der Fahrzeuge entsprechend zu koordinieren.

Falls eine Auswertung der Ausgaben der Sensoren bereits während des Einsatzes vom Computer 15 vorgenommen wird, kann dieser, falls bei der Auswertung ein Fremdkörpers 24 im Schwad 8 erfasst wird, das Gespann 1 anhalten, um dem Fahrer Gelegenheit zu geben, den Fremdkörper 24 in Augenschein zu nehmen und ggf. aus dem Schwad 8 zu beseitigen. Die zum Anhalten notwendige Verzögerung des Gespanns 1 kann klein gehalten werden, da anders als im eingangs zitierten Stand der Technik keine Gefahr besteht, dass der Fremdkörper 24 kurz nach seiner Erfassung etwa von einer Ballenpresse aufgenommen und dadurch unerreichbar wird. Unter Umständen kann der Fremdkörper 24 auch zunächst im Schwad 8 verbleiben, denn wenn der Ort des Fremdkörpers 24 zu Beginn einer zu einem späteren Zeitpunkt stattfindenden Bergungsoperation noch bekannt ist, kann ein landwirtschaftliches Fahrzeug, das die Bergung durchführt, in Kenntnis der Position des Fremdkörpers vor Erreichen dieses Ortes behutsam gestoppt und der Fahrer dann aufgefordert werden, den Fremdkörper zu entfernen. Denkbar ist auch, dass der Fremdkörper beim Bergen gar nicht entfernt wird, sondern dass ein Gutaufnahmewerkzeug des bergenden Fahrzeugs rechtzeitig vor Erreichen des Fremdkörpers 24 zeitweilig angehoben wird, so dass der Fremdkörper 24 zusammen mit umgebendem Material des Schwads 8 auf dem Feld verbleibt. Das zurückgelassene Material mit dem Fremdkörper 24 darin kann dann zu einem späteren Zeitpunkt beseitigt werden.

Die Raumwinkel 21 der beiden an den Enden des Sensorbalkens 9 angebrachten Kameras 10 erfassen auch die Bereiche 13 der Fläche 4 beiderseits des Schwads 8 und eventuell darauf zurückgebliebenes Material 26. Die Dichte dieses Materials 26 in den Bereichen 13 wird vom Computer 15 in Echtzeit ausgewertet, um die Höhe der Kreisel 5 des Schwaders 3 oder 16 daran ausrichten zu können. Wenn diese Dichte auf wenigstens einer Seite des Schwads 8 über einem Grenzwert liegt, veranlasst der Computer 15, dass der Schwader den oder die auf dieser Seite wirkenden Kreisel 5 um eine vorgegebene Schrittweite absenkt, um das Material 26 vollständiger zu erfassen und in den Schwad zu überführen. Führt diese Absenkung dazu, dass der Grenzwert unterschritten wird, so kann die erreichte Höhe des Kreisels 5 beibehalten werden. Eine abrupte Zunahme des vom Nabensensor des betreffenden Kreisels 5 erfassten Drehmoments deutet auf Bodenkontakt des Kreisels 5 hin und veranlasst den Computer 15, den betreffenden Kreisel 5 wieder anzuheben.

### Bezugszeichen

- 1: Gespann
- 2: Traktor
- 3: Schwader
- 4: landwirtschaftliche Fläche
- 5: Kreisel
- 6: Nabe
- 7: Rechen
- 8: Schwad
- 9: Sensorbalken
- 10: Sensor / Kamera
- 11: Sensor /Lidar-Sensor
- 12: Sensor / Radar-Sensor
- 13: Bereich (der Fläche 4)
- 14: Satellitennavigationssystem
- 15: Computer
- 16: Schwader
- 17: Joch
- 18: Welle
- 19: Schwadtuch
- 20: Warnschild
- 21: Raumwinkel
- 22: Oberflächenbereich
- 23: Oberflächenbereich
- 24: Fremdkörper
- 25: Massenspeicher

## Patentansprüche

1. Landmaschine (1) mit einer Schwadsensorik (9-12) zum Erfassen von einer oder mehreren Eigenschaften eines Schwads (8) auf einer von der Landmaschine (1) befahrenen landwirtschaftlichen Fläche (4), **dadurch gekennzeichnet, dass** die Landmaschine (1) eine Vorrichtung zur Schwaderzeugung (3, 16) umfasst und die Schwadsensorik (9-12) wenigstens einen Sensor (10, 11, 12) umfasst, der angeordnet ist, um Eigenschaften eines Stroms von schwadbildendem Material während oder nach seiner Verarbeitung durch die Vorrichtung (3, 16) zu erfassen.

2. Landmaschine nach Anspruch 1, bei der wenigstens ein Sensor (10, 11, 12) der Sensorik auf einen in Fahrtrichtung der Landmaschine (1) hinter der Vorrichtung (3, 16) liegenden Bereich der Fläche (4) ausgerichtet ist, um Eigenschaften eines von der Vorrichtung erzeugten Schwads (8) zu erfassen.

3. Landmaschine nach Anspruch 1 oder 2, bei der der wenigstens eine Sensor ausgewählt ist unter einer Kamera (10), einem Radarsensor (12), einem Lidarsensor (11) oder einem NIR-Sensor.

4. Landmaschine nach Anspruch 1, bei der wenigstens ein Sensor der Sensorik ein Nabensensor oder ein Schwadtuchsensor eines Kreiselschwaders (16) ist.

5. Landmaschine nach einem der vorhergehenden Ansprüche, bei der die Sensorik (9-12) eingerichtet ist, Messdaten der ein oder mehreren Eigenschaften jeweils verknüpft mit einem Ort ihrer Messung zu liefern.

6. Landmaschine nach einem der vorhergehenden Ansprüche, bei der die ein oder mehreren Eigenschaften wenigstens eine von Schwadhöhe, -breite, -querschnitt, Position einer Schwadober- oder -unterseite, Trockenmasse- oder Wasseranteil, Dichte, Homogenität, enthaltene Pflanzenarten betreffen.

7. Landmaschine nach einem der vorhergehenden Ansprüche, bei dem die Schwadsensorik mehrere quer zur Fahrtrichtung (FR) gegeneinander versetzte Sensoren (10, 11, 12) umfasst.

8. Landmaschine nach einem der vorhergehenden Ansprüche, ferner mit wenigstens einem Sensor (10), der angeordnet ist, um wenigstens eine Eigenschaft von zum Schwad seitlich benachbarten Teilen der Fläche zu erfassen, wobei optional die wenigstens eine Eigenschaft eine Konzentration von schwadbildendem Material auf den Teilen der Fläche (4) ist.

9. Landmaschine nach einem der vorhergehenden Ansprüche, bei dem die Sensorik Sensoren (10, 11, 12) unterschiedlicher Typen umfasst.
